(19) Europäisches Patentamt / European Patent Office / Office européen des brevets

(11) **EP 3 775 927 B1**

(12) **EUROPEAN PATENT SPECIFICATION**

(45) Date of publication and mention
of the grant of the patent:
**07.02.2024 Bulletin 2024/06**

(21) Application number: **19776359.2**

(22) Date of filing: **26.03.2019**

(51) International Patent Classification (IPC):
**G01N 33/68** (2006.01)     **G01N 33/49** (2006.01)
**G01N 33/50** (2006.01)

(52) Cooperative Patent Classification (CPC):
**G01N 33/6842;** G01N 2800/52; G01N 2800/7009

(86) International application number:
**PCT/AU2019/050267**

(87) International publication number:
**WO 2019/183671 (03.10.2019 Gazette 2019/40)**

(54) **METHODS FOR MEASURING RELATIVE OXIDATION LEVELS OF A PROTEIN**

VERFAHREN ZUR MESSUNG DER RELATIVEN OXIDATIONSWERTE EINES PROTEINS

MÉTHODES DE MESURE DE TAUX RELATIFS D'OXYDATION D'UNE PROTÉINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.03.2018 AU 2018901026**

(43) Date of publication of application:
**17.02.2021 Bulletin 2021/07**

(73) Proprietor: **Two-Tag Holdings Pty Ltd**
**West Perth WA 6005 (AU)**

(72) Inventors:
• **TAN, Pearl Lin**
**Queens Park, Western Australia 6107 (AU)**
• **ARTHUR, Peter Graeme**
**Wembley, Western Australia 6014 (AU)**
• **LIM, Zi Xiang**
**Singapore 470719 (SG)**

(74) Representative: **Script IP Limited**
**Suite J, Anchor House**
**School Close**
**Chandlers Ford**
**Eastleigh, Hampshire SO53 4DY (GB)**

(56) References cited:
**WO-A1-2007/059567**

• **TAN PEARL LIN ET AL: "Differential thiol oxidation of the signaling proteins Akt, PTEN or PP2A determines whether Akt phosphorylation is enhanced or inhibited by oxidative stress in C2C12 myotubes derived from skeletal muscle",** INTERNATIONAL JOURNAL OF BIOCHEMISTRY AND CELL BIOLOGY, vol. 62, 1 May 2015 (2015-05-01), pages 72-79, XP055853017, AMSTERDAM, NL ISSN: 1357-2725, DOI: 10.1016/j.biocel.2015.02.015
• **BURGOYNE JOSEPH ROBERT ET AL: "The PEG-switch assay: A fast semi-quantitative method to determine protein reversible cysteine oxidation",** JOURNAL OF PHARMACOLOGICAL AND TOXICOLOGICAL METHODS, vol. 68, no. 3, 12 July 2013 (2013-07-12), pages 297-301, XP028759150, ISSN: 1056-8719, DOI: 10.1016/J.VASCN.2013.07.001
• **ANTONIO JUNIOR LEPEDDA ET AL: "Human Serum Albumin Cys34 Oxidative Modifications following Infiltration in the Carotid Atherosclerotic Plaque",** OXIDATIVE MEDICINE AND CELLULAR LONGEVITY, vol. 30, no. 8, 1 January 2014 (2014-01-01), pages 2113-7, XP055223136, US ISSN: 1942-0900, DOI: 10.1155/2014/690953
• **BAR-OR DAVID ET AL: "The cobalt-albumin binding assay: Insights into its mode of ac",** CLINICA CHIMICA ACTA, vol. 387, no. 1, 2 October 2007 (2007-10-02), pages 120-127, XP029159188, ISSN: 0009-8981, DOI: 10.1016/J.CCA.2007.09.018

• BURGOYNE J.R. et al.: "The PEG-switch assay: a fast semi-quantitative method to determine protein reversible cysteine oxidation", J Pharmacol Toxicol Methods, vol. 68, 2013, pages 297-301, XP028759150, DOI: doi:10.1016/j.vascn.2013.07.001

**Description**

**FIELD OF THE INVENTION**

The present invention relates to a method for assessing the oxidation states of a protein in a sample. The invention also relates to a method for detecting protein oxidation, particularly modification caused by reactive oxygen species (ROS) and to kits and other uses of the methods described herein.

**BACKGROUND To THE INVENTION**

In humans, disease and physiological perturbations (e.g. hypoxia, heat, exercise, nutrition) can result in the generation of reactive oxygen species (ROS) which affect cellular function. How ROS affect cellular function depends on their type (e.g. hydroxyl radicals) and cellular location. For example, hydroxyl radicals generated in membranes can initiate peroxidation of lipids and, if severe enough, the resultant membrane leakiness can cause cell necrosis.

As a consequence of interest in the biological effects of ROS, biomarkers have been identified in blood and urine. For example, plasma $F_2$-isoprostanes, a commonly used biomarker of oxidative stress, are lipid degradation products resulting from the actions of highly ROS such as hydroxyl radicals.

Proteins are also targets of highly ROS, such as hydroxyl radicals, which can irreversibly damage proteins with deleterious consequences on protein function. A commonly used plasma assay to detect this type of protein oxidation is the protein carbonyl assay. Carbonyl derivatives are formed directly by ROS, such as hydroxyl radicals, or indirectly by secondary reactions with reactive carbonyl derivatives on carbohydrates.

In addition to irreversible oxidative damage, protein function can be affected by the oxidation of thiol groups of cysteine residues. Oxidation of thiol groups has been shown to affect the function of multiple proteins and has been linked to effects on a range of cellular pathways including proliferation, differentiation, necrosis and contractility. Thiol groups can be oxidised by milder oxidants such as hydrogen peroxide and are also particularly susceptible to oxidation by hypochlorous acid, a ROS produced during inflammatory responses. Accordingly, plasma proteins containing thiol groups are potential biomarkers for protein thiol oxidation. For example, although most thiol groups in plasma proteins are oxidised, the thiol group of cysteine 34 in human serum albumin, is only partially oxidised.

HPLC has been used to separate albumin into three forms based on the oxidation of cys34: a reduced state (-SH); a (reversibly) oxidised state which can convert back to the reduced state (-SOH, -SSX, where X is predominantly cysteine, homocysteine or glutathione); and a biologically irreversible oxidised state (-$SO_2H$, -$SO_3H$). Using HPLC, oxidation of cys34 has been shown to be increased after exercise, aging, haemodialysis patients, chronic kidney disease, diabetes, sleep apnoea and liver cirrhosis.

Although the oxidation state of cys34 appears to be useful for tracking oxidative stress in plasma, the HPLC technique is not widely used. One possible reason for the lack of widespread acceptance is that the analysis requires access to expensive HPLC equipment and associated analytical skills.

D1 (TAN PEARL LIN *et al.,* INTERNATIONAL JOURNAL OF BIOCHEMISTRY AND

CELL BIOLOGY, AMSTERDAM, NL, vol. 62, pages 72 - 79 (doi:10.1016/j.biocel.2015.02.015) discloses that the differential thiol oxidation of the signaling proteins Akt, PTEN or PP2A determines whether Akt phosphorylation is enhanced or inhibited by oxidative stress in C2C 12 myotubes derived from skeletal muscle.

D2 (BURGOYNE JOSEPH ROBERT et al., JOURNAL OF PHARMACOLOGICAL AND TOXICOLOGICAL METHODS, vol. 68, no. 3, pages 297 - 301 (doi:10.1016/J.VASCN.2013.07.001) discusses the PEG-switch assay, which is disclosed as a fast semi-quantitative method to determine protein reversible cysteine oxidation.

D3 (ANTONIO JUNIOR LEPEDDA et al., OXIDATIVE MEDICINE AND CELLULAR LONGEVITY, US, vol. 30, no. 8, pages 2113 - 7 (doi:10.1155/2014/690953) discloses Human Serum Albumin Cys34 Oxidative Modifications following Infiltration in the Carotid Atherosclerotic Plaque.

D4 (BAR-OR DAVID et al., CLINIC A CHIMICA ACTA, vol. 387, no. 1, pages 120 - 127 (doi:10.1016/J.CCA.2007.09.018) discusses the cobalt-albumin binding assay and discloses insights into its mode of action.

It is against this background and the problems and difficulties associated therewith that the present invention has been developed.

**SUMMARY OF THE INVENTION**

The only protection sought is for the invention as claimed.

The present invention is defined in the independent claims. Certain optional features are defined in the dependent claims.

According to a first aspect the present invention provides a method for assessing the oxidation states of a protein in a sample, wherein the protein is a protein selected from the list comprising: albumin, alpha-2-macroglobulin, fibrinogen beta chain, haptoglobin, immunoglobulin lambda constant 2, inter-alpha-trypsin inhibitor heavy chain H2, serotransferrin, immunoglobulin gamma-1 heavy chain, fibrinogen gamma chain and transthyretin, the method comprising the steps of:

(a) contacting the sample with a first label comprising a sulfhydryl-reactive chemical group to selectively bind to a reduced cysteine group of the protein therein to form a first labelled sample;

(b) forming a sub-sample of the first labelled sample;

(c) treating the sub-sample with an effective amount of a thiol containing agent to cause a thiol-disulphide exchange that reduces at least one reversibly oxidised cysteine group of the protein therein preferentially to any irreversibly oxidised cysteine groups therein to form a treated sub-sample;

(d) contacting the treated sub-sample with a second label comprising a sulfhydryl-reactive chemical group to selectively bind to a reduced cysteine group of the protein therein formed during step (c) to form a second labelled sample; and

(e) assessing the first and second labelled samples for a plurality of oxidation states of the protein.

In one embodiment the protein is albumin and the at least one reversibly oxidised cysteine group is a reversibly oxidised cysteine group at cys34.

In one embodiment the first label is further adapted to trap the reduced cysteine group.

In one embodiment the first label is used at a concentration of at least 3mM, 3.6mM, 5mM, 6mM, 6.25mM, 7mM, 8mM, 9mM or 10mM.

In one embodiment the first label is contacted with the sample for at least 5, 10, 15 or 20 minutes.

In one embodiment the first label comprises a fluorescent compound.

In one embodiment the thiol containing agent is adapted to react with the reversibly oxidised cysteine group in a reaction with an equilibrium constant (K) value of between 1 and 2, 1 and 3 or 1 and 4.

In one embodiment the thiol containing agent is selected from the group comprising: cysteine, glutathione (reduced), mercaptoethanol, cysteamine, penicillamine and N-acetylcysteine.

In one embodiment the thiol containing agent is used at a final concentration of at least 2mM, 4mM, 6mM, 8mM, 10mM, 12mM, 12.5mM, 15mM or 20mM.

In one embodiment the thiol containing agent is contacted with the subsample for at least 5, 10, 15, 20 or 30 minutes.

In one embodiment the method further comprises the step of quantifying the amount of the identified oxidation states of the protein.

For sufficiency and completeness, the further information below is provided.

**BRIEF DESCRIPTION OF THE DRAWINGS**

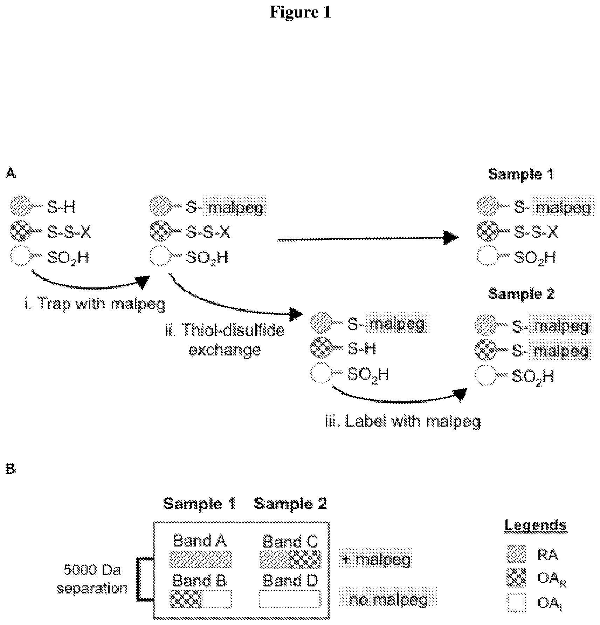

Figure 1 is a diagrammatic representation of the malpeg labelling technique. Ai. Available thiols (-S-H) in the plasma sample are initially trapped with malpeg. Aii. The sample is split in two, with reversibly oxidised thiols (-S-S-X) in the second sample converted to reduced thiols via thiol-disulphide exchange reactions. Aiii. Reduced thiols are labelled with malpeg. B. Following electrophoresis, albumin bound to malpeg is separated by about 5000 Da from unbound albumin. For sample 1, band A represents RA, whereas as band B represents OAR and OAI. For sample 2, band C represents RA and OAR, whereas band D represents OAI.

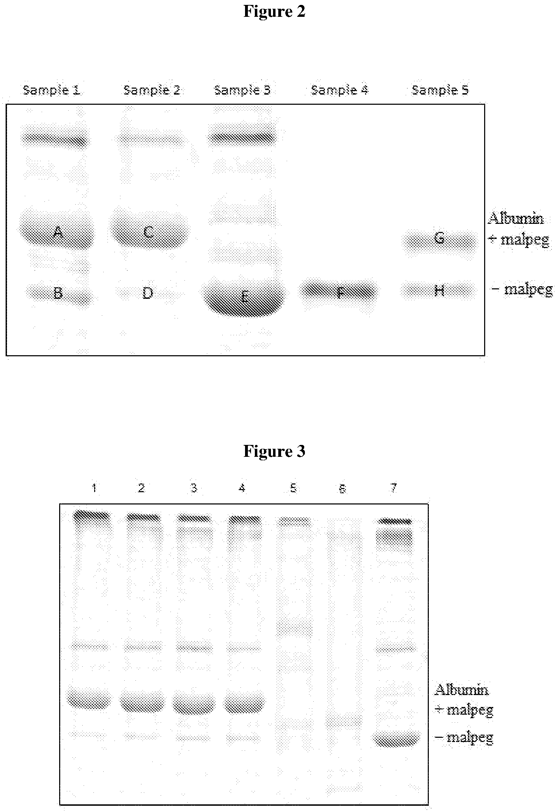

Figure 2 shows the separation of differently oxidised forms of albumin using malpeg. Sample 1. Plasma incubated with malpeg as described in methods (procedure 1). Sample 2. Plasma incubated with malpeg following treatment with cysteine as described in methods (procedure 2). Sample 3. Plasma not incubated with malpeg. Sample 4. Commercial human albumin not incubated with malpeg. Sample 5 Commercial human albumin incubated with malpeg as described in methods (procedure 1). Composition of bands: A, RA; B, $OA_R$ and $OA_i$; C, RA and $OA_R$; D, $OA_i$; E, RA, $OA_R$ and $OA_i$; F, RA, $OA_R$ and $OA_i$; G, RA and $OA_R$; H, $OA_i$.

Figure 3 is an image of a gel showing the shift in the albumin band following incubation of plasma with thiol/disulfide exchange or reducing agents. Albumin was incubated for 30 minutes with concentrations of 10 mM for cysteine (lane 1), glutathione (lane 2), N-acetylcysteine (lane 3), mercaptoethanol (lane 4), DTT (lane 5) and TCEP (lane 6). Following incubation, 12.5 mM malpeg was added for 15 minutes.

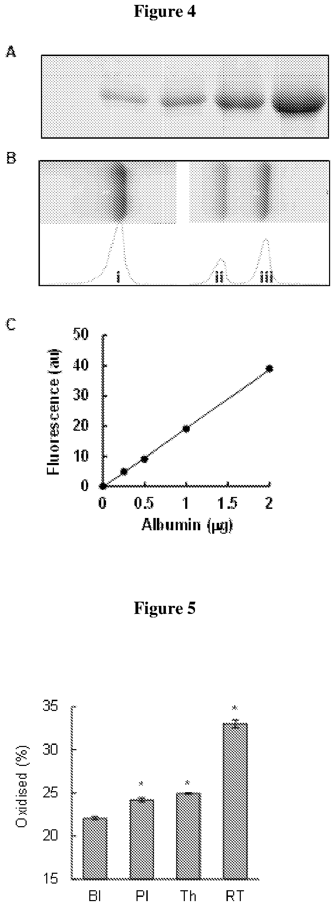

Figure 4 illustrates the use of fluorescent analysis to quantify albumin. Human albumin was loaded on to a gel and proteins were imaged fluorescently (A), quantified (B) and a standard curve was generated (C). The signal profile for the fluorescently imaged gel is shown below the gel image. Bi show albumin in the absence of malpeg, Bii show albumin bound to malpeg, Biii show unlabelled albumin.

Figure 5 is a graph showing the impact of sample preparation and collection - the level of albumin oxidation following treatment with malpeg immediately after sampling blood (BI), immediately after preparation of plasma (PI), immediately after thawing frozen plasma (Th), and after 2.5 hours at room temperature (RT). * represents significantly different from immediately after. Values are expressed in mean $\pm$ SE. (n=3).

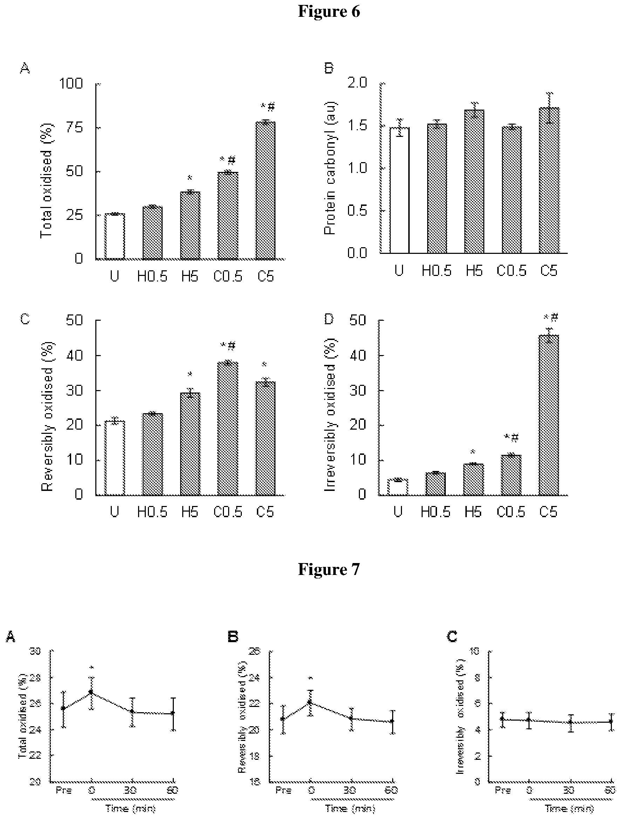

Figure 6 is a series of graphs showing the effect of treatment with hydrogen peroxide or hypochlorous acid on protein oxidation. Plasma samples were untreated (U), treated with 0.5 mM (H0.5) or 5 mM (H5) hydrogen peroxide, 0.5 mM (C0.5) or 5 mM (C5) hypochlorous acid. Levels of (A) total albumin thiol oxidation, (B) protein carbonyl in arbitrary units (au), (C) reversibly oxidised albumin and (D) irreversibly oxidised albumin are shown. * represents significantly different from untreated. # represents significantly different from equivalent concentration of hydrogen peroxide. (n=3-4); and

Figure 7 is a series of graphs showing the effect of exercise on albumin oxidation. Capillary blood samples were collected prior to exercise (Pre), and after exercise to $\dot{V}O_{2Peak}$. Levels of (A) total oxidised albumin, (B) reversibly oxidised and (C) irreversibly oxidised albumin. * represents significantly different from pre-exercise value. Values are expressed in mean $\pm$SE. (n=6)

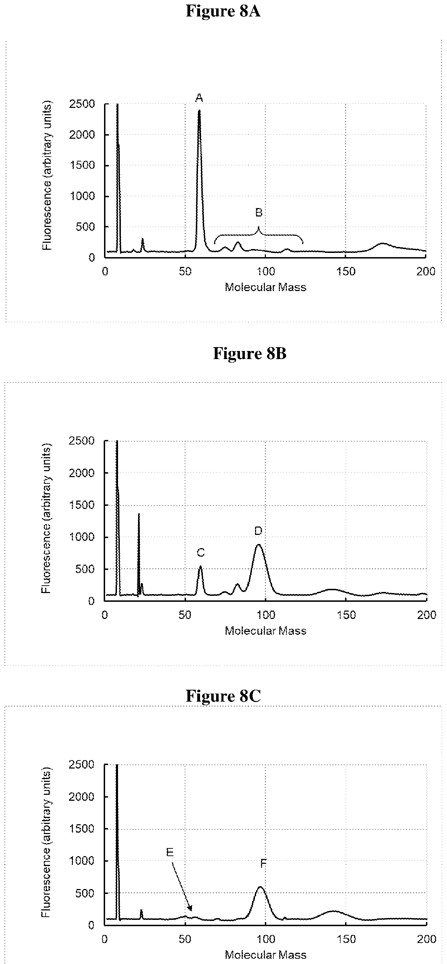

Figure 8 is a series of graphs. Figure 8A showing (i) total albumin (A) and other blood proteins (B) in an untreated sample; Figure 8B showing (ii) oxidised albumin (C) and reduced albumin (D) in a sample treated with malpeg; and Figure 8C showing irreversibly oxidised albumin (E) and reversibly oxidised and reduced albumin (F) in a sample treated with malpgeg and reduced with cysteine. The samples were processed using capillary electrophoresis in accordance with Example 4.

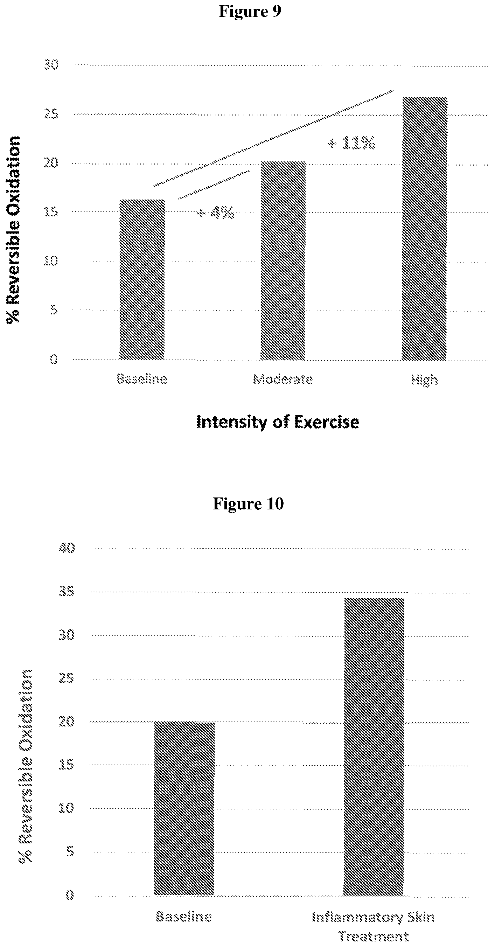

Figure 9 is a graph showing the effect of moderate and high-density exercise on albumin oxidation. Finger prick blood samples were collected on a dried blood spot card embedded with malpeg, prior to and after both moderate and high-density exercise. The graph shows the percentage of reversibly oxidised albumin in the samples.

Figure 10 is a graph showing the effect of an inflammatory skin treatment on albumin oxidation. Finger prick blood samples were collected on a dried blood spot card embedded with malpeg, prior to and after treatment. The graph shows the percentage of reversibly oxidised albumin in the samples.

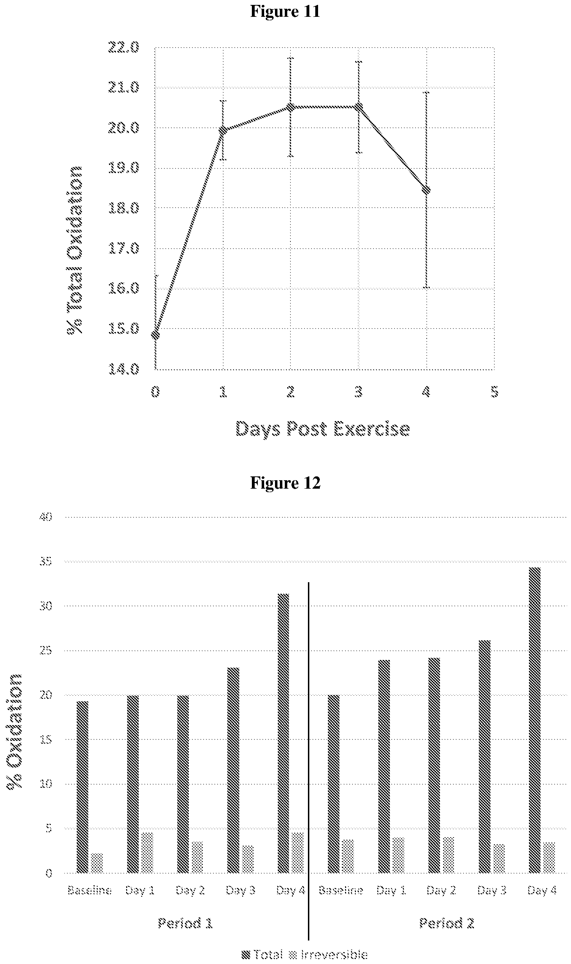

Figure 11 is a graph showing the effect of muscle damage on albumin oxidation in an untrained individual. Finger prick blood samples were collected on a dried blood spot card embedded with malpeg, prior to and for 4 days post weight training. The graph shows the percentage of reversibly oxidised albumin in the samples.

Figure 12 is a graph showing the effect of exerice on both irreversible and reversable albumin oxidation in a patient after both moderate and high intensity exercise over two four-day exercise periods. Finger prick blood samples were collected on a dried blood spot card embedded with malpeg, prior to and after both moderate and high-density exercise periods.

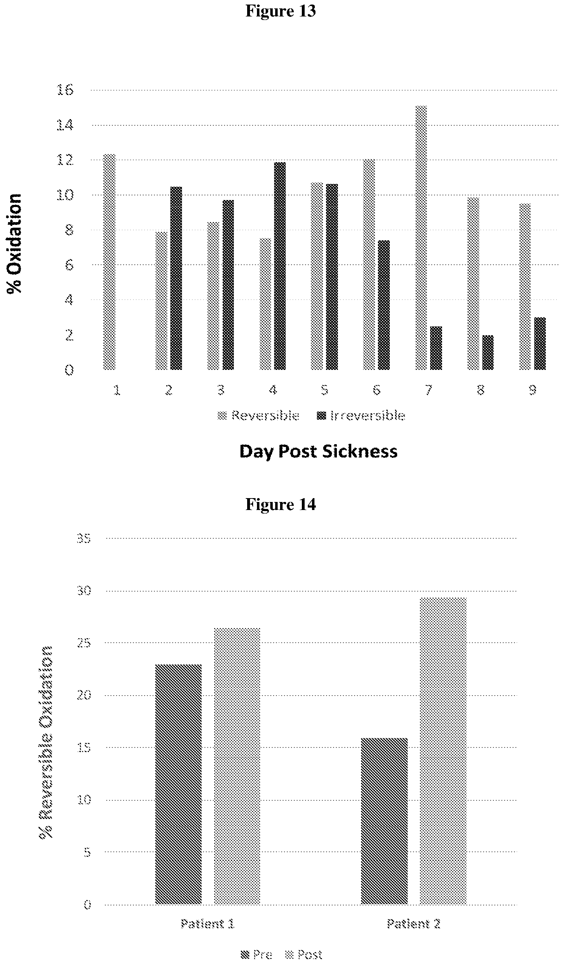

Figure 13 is a graph showing the effect of sickness on albumin oxidation in a patient. Finger prick blood samples were collected on a dried blood spot card embedded with malpeg over a period of 9 days.

Figure 14 is a graph showing the effect of aerobic exercise on reversable albumin oxidation in two patients with different aerobic fitness levels. Finger prick blood samples were collected on a dried blood spot card embedded with malpeg, prior to and after exercise.

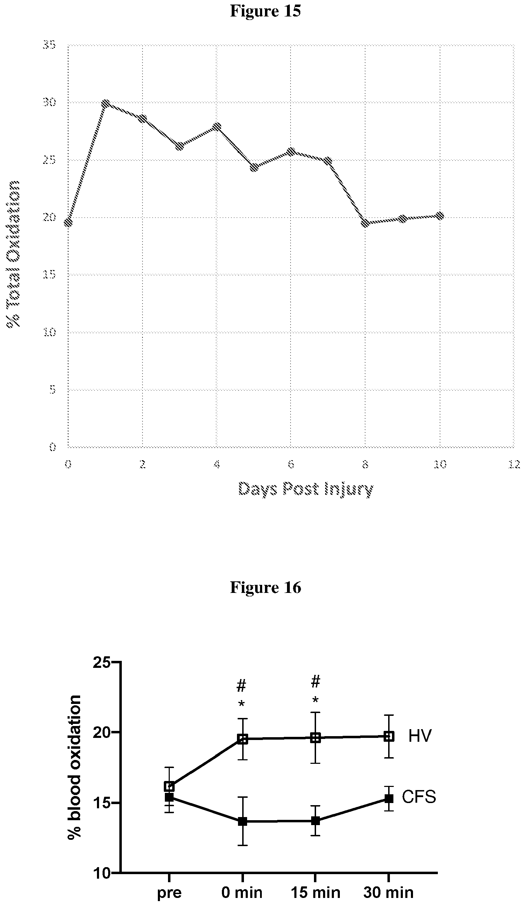

Figure 15 is a graph showing changes in reversible albumin oxidation levels in a patient with a grade 1 calf muscle injury over time. Finger prick blood samples were collected on a dried blood spot card embedded with malpeg, over a period of 10 days post muscle injury.

Figure16 is a graph showing the effect of isometric contraction on protein oxidation level in blood of individuals with CFS (n=12) and healthy sedentary individuals (n=12.

## DETAILED DESCRIPTION OF THE INVENTION

According to a first aspect the present invention provides a method for assessing the oxidation states of a protein in a sample, wherein the protein is a protein selected from the list comprising: albumin, alpha-2-macroglobulin, fibrinogen beta chain, haptoglobin, immunoglobulin lambda constant 2, inter-alpha-trypsin inhibitor heavy chain H2, serotransferrin, immunoglobulin gamma-1 heavy chain, fibrinogen gamma chain and transthyretin, the method comprising the steps of:

(a) contacting the sample with a first label comprising a sulfhydryl-reactive chemical group to selectively bind to a reduced cysteine group of the protein therein to form a first labelled sample;

(b) forming a sub-sample of the first labelled sample;

(c) treating the sub-sample with an effective amount of a thiol containing agent to cause a thiol-disulphide exchange that reduces at least one reversibly oxidised cysteine group of the protein therein preferentially to any irreversibly oxidised cysteine groups therein to form a treated sub-sample;

(d) contacting the treated sub-sample with a second label comprising a sulfhydryl-reactive chemical group to selectively bind to a reduced cysteine group of the protein therein formed during step (c) to form a second labelled sample; and

(e) assessing the first and second labelled samples for a plurality of oxidation states of the protein.

Preferably, the oxidation states comprise a reversibly oxidised form.
Preferably, the oxidation states comprise an irreversibly oxidised form.
Preferably, the oxidation states comprise a reversibly and an irreversibly oxidised form.
When the protein is albumin and the oxidation state comprises a reversibly oxidised form, the reversibly oxidised form preferably comprises a reversibly oxidised cysteine group at cys34.
Preferably, the protein is an animal protein such as a fish or mammalian protein. Even more preferably, the protein is a human protein.
Preferably, the sample is a body fluid sample such as a mammalian, preferably human, body fluid sample. More preferably, the sample is selected from the list of samples comprising: blood, blood plasma, blood serum, urine, milk and saliva. The sample may also be a cell extract or some other preparation derived from biological material such as a tissue sample or extract thereof. The sample can also be part of a cell such as a sample containing mitochondria or another subcellular organelle.
The sample may also comprise a single protein or a plurality of proteins. When the sample comprises a plurality of proteins the method of the present invention can be used to assess the oxidation states of the plurality of proteins in the sample. For example, the method can be used to produce a profile that indicates which proteins in a sample have been oxidised and which ones have not.
Preferably, the first label is further adapted to trap the reduced cysteine group such that the bond formed between the label and the reduced cysteine group cannot be cleaved with a reducing agent.
When the first label is adapted to trap the reduced cysteine group it is preferably contacted with the sample as soon as possible after the sample is taken. For example, the first label may be contacted with the sample less than 1, 2, 3, 4 or 5 minutes of the sample being taken. In this regard, applicant has surprisingly discovered that the handling of a sample prior to assessing the oxidation states of proteins therein can impact on the accuracy of any assessment of oxidation states.
Preferably, the first label comprises a sulfhydryl-reactive chemical group. Even more preferably, the first label comprises a maleimide group; a haloacetyl group, such as an iodoacetyl or a bromoacetyl group; and/or a pyridyl disulphide group.
The first label may be selected from the group consisting of: maleimide, phenyl mercury, iodoacetamide, vinylpyridine, methyl bromide or iodoacetate or derivatives thereof. Preferably, this component is iodoacetamide or maleimide or a derivative thereof.

Preferably, the first label is used at a concentration of at least 3mM, 3.6mM, 5mM, 6mM, 6.25mM, 7mM, 8mM, 9mM or 10mM for at least 5, 10, 15 or 20 minutes when contacted with the sample.

Preferably, the first label further comprises a separation member adapted to facilitate separation of a labelled compound relative to unlabelled compounds.

The separation member may be a compound with a defined molecular weight that facilitates separation based on weight differences. Even more preferably, the separation member is a polymer such as polyethylene glycol. Thus, for example, the first agent may be pegylated.

The separation member may also be a fluorescent compound capable of being imaged.

The first label may also be a mass tag or label that facilitates identification via mass spectrometry or another similar methodology. Examples of suitable mass tags include: biotin-maleimide, iodoacetamide and N-Ethylmaleimide.The first label may also be an antigen.

Preferably, the sub-sample comprises a volume of about 50% of the volume of the labelled sample.

Preferably, treating the sub-sample to selectively reduce at least one reversibly oxidised cysteine group of the protein therein comprises the step of contacting the sub-sample with an effective amount of a thiol containing agent. Preferably, the thiol containing agent is adapted to react with the reversibly oxidised cysteine group in a thiol-disulphide exchange reaction that only slightly or moderately favours reduction of the reversibly oxidised cysteine group. In this regard, the thiol containing agent may be adapted to react with the reversibly oxidised cysteine group in a reaction with an equilibrium constant (pKa) value of: less than 8 or 9 and more preferably less than 4, 5, 6, or 7.

Preferably, the thiol containing agent comprises a compound including a single thiol group.

Preferably, the thiol containing agent is selected from the group comprising: cysteine, glutathione (reduced), mercaptoethanol, cysteamine, penicillamine and N-acetylcysteine.

For the purposes of the present invention, the term "selectively" when used in the phrase "selectively reduce" means that reversibly oxidised cysteine groups are reduced preferentially to any irreversibly oxidised cysteine groups in the sample. Preferably, the term "selectively reduce" means that there is no measurable reduction of any irreversibly oxidised cysteine groups in the sample. In one particular form of the invention the term "selectively reduce" means that only a subset of reversibly oxidised cysteine groups is reduced e.g. for albumin, cysteine residue 34 is selectively reduced relative to other reversibly oxidised cysteine groups in albumin.

Preferably, the thiol containing agent is used at a final concentration of at least 2mM, 4mM, 6mM, 8mM, 10mM, 12mM, 12.5mM, 15mM or 20mM.

Preferably, the thiol containing agent is contacted with the subsample for at least 5, 10, 15, 20 or 30 minutes.

Preferably, the second label is further adapted to trap the reduced cysteine group such that the bond formed there between cannot be cleaved with a reducing agent.

Preferably, the second label comprises a sulfhydryl-reactive chemical group. Even more preferably, the second label comprises a maleimide group; a haloacetyl group, such as an iodoacetyl or a bromoacetyl group; and/or a pyridyl disulphide group.

Preferably, the second label further comprises a separation member as described herein in relation to the first label.

Preferably, the second label has the same reaction chemistry/binding characteristics as the first label.

Preferably, the second label is distinguishable from the first label. For example, the second label may incorporate a different antigen, mass, absorbance or fluorescent tag.

Preferably, the second label is used at a concentration that is higher than that used for the first label. Even more preferably, the concentration of the second label may be at least 5mM, 7.5mM, 10mM, 12.5mM or 15mM for at least 5, 10, 15 or 20 minutes when contacted with the treated sub-sample.

The step of assessing the first and second labelled samples for a plurality of oxidation states of the protein will vary depending at least in part on the choice of the first and second label. Preferably, the step of assessing comprises applying the first and second labelled samples to size based separation such as electrophoresis.

Preferably, the method of the present invention is quantitative. Thus, the method may further comprise the step of quantifying the amount of the identified oxidation states of the protein.

Preferably, the oxidation states are quantified in relative terms.

Preferably, the oxidation states are quantified as a percentage, such as a percentage of the total amount of the protein in the sample. Preferably, the oxidation states are quantified as a percentage of the protein that is oxidised.

Preferably, the oxidation states are quantified by reference to the intensity of a signal from the first or second label.

One particularly useful means for assessing the first and second labelled samples is gel electrophoresis such as PAGE as the protein sample can be applied to PAGE and then the signals from the labels measured at particular protein bands on the gel. Another suitable technique for assessing the first and second labelled samples is capillary electrophoresis, a high-speed protein analysis technique which uses the same principle of separation as PAGE electrophoresis but is performed in a gel or polymer filled capillary tube. Dependent on the type of label, other visualising means include immunoblotting, phospho-imaging or lumi-imaging.

Alternate techniques to PAGE are immunoprecipitation or lateral flow strips (where a single protein of interest is isolated), protein or antibody arrays (where a multitude of proteins are isolated on a protein chip), and mass spectrometry and/or chromatography, where single or total protein extracts are analysed (for example by multidimensional chromatography). Mass spectrometry and the protein or antibody arrays offer the opportunity to scan 10, 100 or even 1000s of proteins very rapidly very much like microarrays.

The method of the present invention may be conveniently performed using a kit comprising a series of reagents necessary to carry out the method. Thus, also described for sufficiency and completeness is a kit for assessing the oxidation states of a protein in a sample, the kit comprising:

(a) a first label adapted to selectively bind to a reduced cysteine group of the protein in the sample; and

(b) a reagent for selectively reducing at least one reversibly oxidised cysteine group of the protein in the sample.

Preferably, the kit further comprises a second label adapted to selectively bind to a reduced cysteine group of the protein formed by treatment with the reagent. Preferably, the first and second label are the same.

Preferably, the kit further comprises a substrate for receiving the sample, Preferably, the substrate comprises the first label, and is adapted to bind at least one reduced cysteine group of the protein from a whole blood sample. Preferably the substrate is an absorbent paper, such as filter paper. Preferably the substrate further comprises at least one sample identifier.

Preferably the kit further compries a sample collection device. Preferably the sample collection device is adapted to enable collection of a capillary blood sample. Preferably the sample collection device is a skin pricking device. Prefably the sample collection device is a hand-held device adapted to enable collection of a capillary blood sample from a heel, finger or ear lobe of a patient. Preferably the sample collection device is a lancet.

Preferably the substrate is a dried blood spot card, such as a Perkin Elmar 226 Spot Saver Card. Preferably the first label is embedded in the dried blood spot card. Preferably the sample is a whole blood sample, such as a finger prick sample. Alternatively, the substrate comprises a separation membrane for separating one or more proteins in a sample from other whole blood compoentns, such as red blood cells.

Preferably, the kit further comprises an extraction reagent adapted to extract at least a portion of the blood sample from the dried blood spot card. Preferably, the kit further compises a protein isolation reagaent adapted to separate the bound protein from the sample.

Preferably, the kit further comprises instructions to utilise the reagents therein according to the methods described herein.

## General

Those skilled in the art will appreciate that the invention described herein is susceptible to variations and modifications other than those specifically described.

The specific embodiments or examples described herein are intended for the purpose of exemplification only, not to limit the invention - the invention is limited as defined in the claims.

Throughout this specification, unless the context requires otherwise, the word "comprise", or variations such as "comprises" or "comprising", will be understood to imply the inclusion of a stated integer or group of integers but not the exclusion of any other integer or group of integers.

Other definitions for selected terms used herein may be found within the detailed description of the invention and apply throughout. Unless otherwise defined, all other scientific and technical terms used herein have the same meaning as commonly understood to one of ordinary skill in the art to which the invention belongs.

## EXAMPLES

The following methods serve to more fully describe the manner of using the above-described invention, as well as to set forth the best modes contemplated for carrying out various aspects of the invention. It is understood that these methods are presented for illustrative purposes only, not to limit the invention - the invention is limited as defined in the claims.

**Example 1 - Quantitative assessment of albumin thiol oxidation after exercise.**

**1. Materials/Methods**

**(a) Participants**

Healthy adults, aged 18-32, participated, with ethics approved by the human ethics committee of The University of Western Australia.

**(b) Materials**

Double-deionized (DDI) water was used throughout. Protein molecular weight standards were purchased from Bio-Rad (Australia). Unless otherwise stated, all chemicals and reagents were obtained from Sigma-Aldrich (Castle Hill, Australia). Polyethylene glycol maleimide (malpeg), 5000 g/mol was purchased from JenKem Technology (USA).

**(c) Blood sample preparation and storage**

For the analysis of albumin thiol oxidation, nine parts of blood was collected into a $K_3$EDTA tube (Minicollect tubes $K_3$EDTA; Greiner Bio-One, Austria), containing 1 part of the trapping solution made up of 62.5 mM malpeg, 40 mM imidazole and 154 mM NaCl diluted in DDI water, pH 7.4. Additional blood was collected into a second $K_3$EDTA tube without trapping solution for the analysis of total plasma albumin. The tubes were briefly vortexed and then centrifuged (3000g, 10 min), and plasma collected. Plasma without trapping solution was immediately frozen in liquid nitrogen and stored at -80°C, whereas plasma with trapping solution was incubated at room temperature for 20 minutes and then frozen and stored.

For the identification of human albumin with and without malpeg on the gel, 0.9 mM commercial human serum albumin (HSA; Sigma) was prepared in SDS/Tris buffer, containing 0.5% (w/v) SDS and 0.5 mM Tris (pH 7.4). Nine parts of HSA sample was added to 1 part of a trapping solution made up of 62.5 mM polyethylene glycol maleimide (Malpeg, 5000 g/mol, JenKem Technology,USA), 40 mM imidazole and 154 mM NaCl diluted in DDI water, pH 7.4. Samples without the trapping solution was immediately frozen in liquid nitrogen and stored at -80 °C, whereas plasma collected in the presence of the trapping solution (Malpeg) was incubated at room temperature for 30 minutes prior to being frozen and stored

**(d) Sample preparation**

Plasma or HSA samples containing malpeg were thawed at 37°C with agitation and then divided into two, 2.5μl aliquots.

Procedure I involved adding SDS/Tris buffer (245 μl) containing 0.5% SDS and 0.5 mM Tris (pH 7.4) to aliquot 1 (Sample 1; Fig 1a).

Procedure II involved adding 2.5μl of 20mM L-cysteine (pH 3) to aliquot 2, incubating for 30min at room temperature, and then adding 5 μl of 25 mM malpeg with a further incubation for 15 minutes at room temperature. A sub-aliquot (4μl) was added to 95 μl of SDS/Tris buffer (Sample 2; Fig 1a).

**(e) Gel electrophoresis**

Gels were hand casted using mini-protean plates (Bio-Rad). Briefly, the 16% resolving gel was made as per the Laemmli method [1]. For fluorescent imaging, 1% (v/v) of 2,2,2-trichloroethanol [2] was added. After the resolving gel was polymerised, the 4% stacking gel was poured on top of the resolving gel and after polymerization, the gels were stored in a dark cold room at least 3 hours before use.

Samples (5 μl of sample 1 and 5 μl sample 2) were mixed with equal parts of loading buffer containing 0.5M TRIS (pH 6.8), 3% (w/v) SDS, 30% (v/v) glycerol and 0.03% (w/v) bromophenol blue in DDI water. A 5μl aliquot was loaded onto gels and gels were run at 250 V for 1 hr 45 mins in the cold and dark room. Following electrophoresis, the gel was washed twice with DDI water. The gel was placed on a UV transilluminator (ChemiDoc™, Biorad) for 5 min and then visualised with Image Lab™ software, Biorad. Images of the gels were analysed using NIH Imaged software [Version 1.48v; [3]]. The image was inverted, and after background subtraction, and editing for speckling and noise a signal profile was plotted for each lane from the gel (Image J user guide 1.46r, 2012). The area under each peak were calculated using the trapezoid rule to give the intensity for each band [2].

(f) **Protein carbonyl assays**

Carbonyl groups formed on plasma albumin were determined with immunoblotting, which involved the processing of samples with positive and negative controls. Plasma samples were diluted 1:120 with 6% SDS. The positive control sample was incubated 1:1 with 50 mM HOCl for 1 hour, then diluted 1:60 with 6% (w/v) SDS. One part of diluted sample or positive control were added to one part of 10 mM dinitrophenyl hydrazine (10 mM DNPH/10% (w/v) TFA). The negative control sample was incubated with the same conditions of 10% (w/v) TFA, but without DNPH. After 15 minutes of incubation, one part of neutralization solution (30% glycerol/ 2 M Tris) was added to the DNPH and negative control treated samples. The treated samples were then diluted 10 times under reducing conditions, with 5 $\mu$l of treated samples separated by stain free SDS-PAGE (Biorad, 4-20% Mini-PROTEAN® TGX Stain-Free™ Precast Gels) and transferred (Biorad, Trans-Blot® Turbo™ Transfer System) onto nitrocellulose membrane (Biorad, Trans-Blot® Turbo™ Mini Nitrocellulose Transfer Packs) using conditions set at 2.5 A, up to 25 V for 10 minutes.

The membrane was subsequently washed in Tris-Buffered Saline Tween 20 (TBST) 5 times, for 3 minutes each (5 x 3 mins), and blocked with TBST/0.5% (w/v) non-fat dry milk. After one hour, the membrane was washed in TBST (5 x 3 mins), then incubated in polyclonal rabbit anti-DNP antibody (diluted 1:20000 in TBST/0.5% (w/v) non-fat dry milk). After an overnight incubation in the cold room, the membrane was washed in TBST (5 x 3 mins) and then treated with horseradish peroxidase-conjugated goat anti rabbit IgG (diluted 1:25000 in TBST/0.5% non-fat dry milk) for 1 hour at room temperature. A final wash with TBST (5 x 3mins) was performed prior to visualisation of carbonylated albumin using ECL western blot detection reagent (Bio-rad, Clarity Western ECL substrate).

Albumin carbonylation was tabulated as a ratiometric value, using the carbonyl density divided by the amount of fluorescence signal of albumin from the stain free gel (loading control). That is: ratio = arbitrary amount of carbonylated albumin/arbitrary amount of albumin. The inter-assay coefficient of variation (standard deviation ÷ mean) for albumin carbonyl was 8.6% (n = 9), similar to previous carbonyl methods with a coefficient of variations ranging from 7%-18% (Dayhoff-Brannigan et al. 2008 [5]; Matthaiou et al. 2014 [6]).

### (i) Statistical analysis

All data are presented as means ± SE unless otherwise states. Means were compared using a t-test or one-way ANOVA with repeated measures where appropriate. Significance was accepted at $p<0.05$.

## 2. Results

### (a) Method development

The quantitative analysis of plasma albumin thiol oxidation state involves maleimide labelling of cys-34 with malpeg, with labelled albumin then separated from unlabelled albumin by SDS-PAGE (Fig. 1 & 2). In particular, Sample 1 was used for analysing the percentage of reduced (RA) and oxidised albumin (OA; Fig 1b). Sample 2 was used to calculate the percentage of albumin in the reversibly oxidised form (OAR) and irreversibly oxidised form (OAI; Fig 1b). For sample 1, the top band (A in Fig. 2) was reduced albumin (RA) and the bottom band (B in Fig. 2) was reversibly and irreversibly oxidised albumin (OAR & OAI). For sample 2, the top band (C in Fig. 2) was reduced (RA) and reversibly oxidised albumin (OAR) and the bottom band (D in Fig. 2) was irreversibly oxidised albumin (OAI). The percentage of albumin in the different forms was calculated as follows:

1.

The percentage of reduced albumin (%RA) = band A/ (band A + band B) * 100

2.

The percentage of irreversibly oxidised albumin (%OAI) = band D / (band D + band E) * 100

3.

$$\text{The percentage of reversibly oxidised albumin (\%OAR)} = 100 - \%RA - \%OAI$$

The technique depends on labelling of the thiol of cys34 by malpeg. A concentration of 6.25 mM malpeg incubated for at least 15 minutes at room temperature was deemed sufficient for maximum labelling.

To measure reversibly oxidised cys34, a thiol-disulfide exchange reaction was used to generate a thiol on cys34 which could then be labelled with malpeg. Cysteine, reduced glutathione, N-acetylcysteine and mercaptoethanol were suitable thiol-disulfide exchange reagents, but dithiothreitol and TCEP were not (Fig. 3). Cysteine was used as a thiol-disulfide exchange reagent in all subsequent experiments. A cysteine concentration of at least 10 mM incubated for at least 15 minutes was sufficient to account for more maximal labelling of the reversibly oxidised thiol. After incubating with cysteine, incubating for at least 15 minutes with 12.5 mM of malpeg was sufficient for labelling of newly exposed thiol groups in cys34.

To quantify the relative oxidation of albumin fluorescent imaging of protein reacted with 2,2,2-trichloroethanol was used to achieve complete separation (Fig. 4bii, iii). There was a linear relationship between albumin content and gel band density (Fig. 4) for fluorescent imaging up to an albumin loading of 2 $\mu$g. This linear relationship meant that the relative fluorescent intensities for albumin bound and not bound to malpeg could be used to calculate relative oxidation. Consistent with this concept the fluorescent intensity of albumin with no malpeg added (22.5 ± 0.7, arbitrary units, n=4) was comparable to the summed fluorescent intensity of sample 1 (22.7 ± 0.5, arbitrary units, n=4) and sample 2 (22.5 ± 0.6, arbitrary units, n=4) which contained both albumin bound and not bound to malpeg. The calculated oxidation of plasma albumin using the fluorescent imaging technique was reproducible, with a intra and inter assay coefficient of variation of 2.7% (n=12) and 4.7% (n=12) respectively.

**(b) Collection and preparation of plasma sample.**

Protein thiol groups are sensitive to oxidation, so there is potential for artefactual oxidation during sample preparation. However, reacting the thiol group of cys34 with malpeg prevents oxidation. Three sample preparation techniques were tested, with malpeg added: to blood as soon as it was collected; to plasma following centrifugation; to freshly thawed plasma; and to plasma after 2.5 hours at room temperature. For all plasma samples, there was increased oxidation relative to the level of albumin oxidation in the blood sample to which malpeg had been added (Fig. 5).

**(c) Comparison with chromatography technique**

Chromatography based techniques have been used to measure fraction of albumin in the oxidised form. Using a chromatography technique described by Turell et al [4], bovine serum albumin samples were estimated to be 36 ± 0.7% (n=5) oxidised, whereas the level of oxidation was estimated to be 42 ± 0.1% (n=5) using the malpeg technique. Because of the discrepancy between the two measurements, bovine serum albumin samples were treated with hydrogen peroxide was used to completely oxidise thiol groups. Using the chromatography, albumin samples were 68 ± 1.8% (n=5) oxidised, whereas the level of oxidation was estimated to be 98 ± 0.1% (n=5) using the malpeg technique. These observations suggest the chromatography technique of Turell et al. underestimated the extent of albumin oxidation.

**(d) Comparison of albumin oxidation method with the protein carbonyl assay**

The sensitivity of the albumin oxidation method was compared to the protein carbonyl assay using two reactive oxygen species, hydrogen peroxide and hypochlorous acid. For hydrogen peroxide, concentrations of 0.5 mM and 5 mM caused significant increases in albumin Cys34 oxidation with no significant increases in protein carbonyl formation (Fig. 6). A similar pattern of oxidation was evident for hypochlorous acid, with a significant increase in albumin Cys34 oxidation, but no significant increase in protein carbonyl formation (Fig. 6A & 6B). At equivalent concentrations, hypochlorous acid caused greater oxidation of albumin than hydrogen peroxide .

Both hydrogen peroxide and hypochlorous acid caused increases in reversibly and irreversibly oxidised albumin (Fig. 6C). However, hypochlorous acid at 5 mM caused a significantly lower increase in reversibly oxidised albumin than at 0.5 mM. This apparent discrepancy is addressed in the discussion.

**(e) Applications: quantitative assessment of human albumin thiol oxidation after exercise.**

The sensitivity of the gel based method assay was tested by measuring human plasma albumin thiol oxidation after exercise. Participants performed a $\dot{V}O_{2Peak}$ stationary cycling exercise test at an initial intensity of 50 watts, with the intensity increasing by 30 watts at 3 mins interval until volitional exhaustion or until the participant was unable to successfully maintain the required power output. Capillary blood samples were collected prior to and after exercise.

Immediately after exercise, there was an increase in oxidised albumin which returned to pre-exercise levels by 30 mins post-exercise (Fig. 7). The increase in oxidised albumin was a consequence of an increase in reversibly oxidised albumin and not irreversibly oxidised albumin (Fig 7).

**Example 2** - **Protein oxidation levels in individuals with Chronic Fatigue Syndrome**

**1. Materials/Methods**

**(a) Participants**

Healthy volunteers (n=12) and people suffering from Chronic Fatigue Syndrome (n=12) performed 3 maximal intensity voluntary contractions involving a unilateral knee extension on an ergometer for 30s, with 120s of recovery between extensions. The knee angle was fixed at 70 degrees.

Blood samples were taken immediately before exercise, immediately after exercise, 15min after exercise, and 30min after exercise.

Participants were required to abstain from consuming alcohol, caffeine, and painkillers for 48 hours before testing. They were required to fast from 10pm the day prior to testing. All testing began at 9am.

**(b) Materials**

Double-deionized (DDI) water was used throughout. Protein molecular weight standards were purchased from Bio-Rad (Australia). Unless otherwise stated, all chemicals and reagents were obtained from Sigma-Aldrich (Castle Hill, Australia). Polyethylene glycol maleimide (malpeg), 5000 g/mol was purchased from JenKem Technology (USA).

**(c) Blood sample preparation and storage**

For the analysis of albumin thiol oxidation, nine parts of blood was collected into a $K_3$EDTA tube (Minicollect tubes $K_3$EDTA; Greiner Bio-One, Austria), containing 1 part of the trapping solution made up of 62.5 mM malpeg, 40 mM imidazole and 154 mM NaCl diluted in DDI water, pH 7.4. Additional blood was collected into a second $K_3$EDTA tube without trapping solution for the analysis of total plasma albumin. The tubes were briefly vortexed and then centrifuged (3000g, 10 min), and plasma collected. Plasma without trapping solution was immediately frozen in liquid nitrogen and stored at -80°C, whereas plasma with trapping solution was incubated at room temperature for 20 minutes and then frozen and stored.

For the identification of human albumin with and without malpeg on the gel, 0.9 mM commercial human serum albumin (HSA; Sigma) was prepared in SDS/Tris buffer, containing 0.5% (w/v) SDS and 0.5 mM Tris (pH 7.4). Nine parts of HSA sample was added to 1 part of a trapping solution made up of 62.5 mM polyethylene glycol maleimide (Malpeg, 5000 g/mol, JenKem Technology,USA), 40 mM imidazole and 154 mM NaCl diluted in DDI water, pH 7.4. Samples without the trapping solution was immediately frozen in liquid nitrogen and stored at -80 °C, whereas plasma collected in the presence of the trapping solution (Malpeg) was incubated at room temperature for 30 minutes prior to being frozen and stored.

**(d) Sample preparation**

Plasma or HSA samples containing malpeg were thawed at 37°C with agitation and then divided into two, 2.5μl aliquots.

Procedure I involved adding SDS/Tris buffer (245 μl) containing 0.5% SDS and 0.5 mM Tris (pH 7.4) to aliquot 1 (Sample 1; Fig 1a).

Procedure II involved adding 2.5μl of 20mM L-cysteine (pH 3) to aliquot 2, incubating for 30min at room temperature, and then adding 5 μl of 25 mM malpeg with a further incubation for 15 minutes at room temperature. A sub-aliquot (4μl) was added to 95 μl of SDS/Tris buffer (Sample 2; Fig 1a).

**(e) Gel electrophoresis**

Gels were hand casted using mini-protean plates (Bio-Rad). Briefly, the 16% resolving gel was made as per the Laemmli method [1]. For fluorescent imaging, 1% (v/v) of 2,2,2-trichloroethanol [2] was added. After the resolving gel was polymerised, the 4% stacking gel was poured on top of the resolving gel and after polymerization, the gels were stored in a dark cold room at least 3 hours before use.

Samples (5 μl of sample 1 and 5 μl sample 2) were mixed with equal parts of loading buffer containing 0.5M TRIS (pH 6.8), 3% (w/v) SDS, 30% (v/v) glycerol and 0.03% (w/v) bromophenol blue in DDI water. A 5$\mu$l aliquot was loaded onto gels and gels were run at 250 V for 1 hr 45 mins in the cold and dark room. Following electrophoresis, the gel was washed twice with DDI water. The gel was placed on a UV transilluminator (ChemiDoc™, Biorad) for 5 min and then visualised with Image Lab™ software, Biorad. Images of the gels were analysed using NIH Imaged software [Version 1.48v; [3]]. The image was inverted, and after background subtraction, and editing for speckling and noise a signal profile was plotted for each lane from the gel (Image J user guide 1.46r, 2012). The area under each peak were calculated using the trapezoid rule to give the intensity for each band [2].

## 2. Results

Figure 16 shows the effect of isometric contraction on protein oxidation level in blood of individuals with CFS (n=12) and healthy sedentary individuals (n=12). Blood was collected pre-exercise (pre), and at 0, 15 and 30 minutes after exercise. Data are shown as mean, with error bars indicating standard errors of the mean. * indicates significant difference (p<0.05) between healthy and CFS participants. # indicates significance (p<0.05) from pre-exercise measurement.

The healthy participants experienced a post-contraction increase in the oxidation state of plasma albumin Cys34. A similar increase was not seen in participants with known Chronic Fatigue Syndrome. Results showed that there was an abnormal response to repeated isometric contraction in the blood of individuals with Chronic Fatigue Sydnrome.

## Example 3 - Effect of various stressors on reversibly oxidized albumin levels

### 1. Materials/Methods

### (a) Materials

Double-deionized (DDI) water was used throughout. Protein molecular weight standards were purchased from Bio-Rad (Australia). Unless otherwise stated, all chemicals and reagents were obtained from Sigma-Aldrich (Castle Hill, Australia). Polyethylene glycol maleimide (malpeg), 2000 g/mol was purchased from JenKem Technology (USA). Perkin Elmar 226 Protein save 5 Spot Cards were used.

### (b) OxiMetric Dried Blood Spot Card Preparation Procedure

100 $\mu$L of 40mM Imidazole was added to 12.5 mg of Methoxy polyethylene glycol in a 1.5mL microfuge tube. The tube was vortexed for approximately 2 minutes. The resulting trapping agent was a clear solution with a final methoxy polyethelene glycol concentration of 62.5 mM.

5 $\mu$L of the prepared trapping agent was pippeted onto the center of each of the 5 spots on a blood card. The trapping agent spread out to cover apprxomately ¾ of the designated circle area of each of the blood spots. The blood card impregnated with trapping agent was placed into the supplied airtight container with dessicant, allowed to dry for at least 2 hours and stored in the same container until required for use.

### (c) Blood Card and Finger Prick Sampling

A blood card comprising the trapping agent was removed from the desiccant container and place on a flat surface, with circles facing up. The container was resealed. A lancet was prepared, by removing the lance cap. The collection site was rubbed for approximately 20 seconds before lancing, The lancet was placed firmly against the puncture site, and the release button was pressed to puncture the skin. The puncture site was gently squeezed to produce a blood drop. One or two blood drops were applied to the center of a circle of the blood card. The sample was lablled with date, time and sample identifier. The top portion of the card was folded and tucked over the collected sample spots and the card was returned to the desiccant container.

The Blood Card can be stored at room temperature for several months, in the provided dessicant container. The dessicant container should be changed if the dessicant changes colour from orange to blue.

### (d) Cibracron Blue Albumin Isolation Method and Thiol Oxidation Analysis

Blood extraction from Blood Card

A 4.5 mm hole was punched through the centre of each spot on each the blood card. Each 4.5mm blood card disk was placed into a separate well of a 96 well plate. 100 µl of 20mM phosphate buffer, 0.05% Tween 20 (pH 7.1) was added to each well containing a blood card disk. The plate was incubated at room temperature on a plate mixer for 2 hours.

Reduction with Cysteine for Reversable Oxidation Analysis

A 40 µL aliquot was transferred from each well containing a blood card sample into a 0.5 mL microfuge tube. A 10 mM cysteine solution was prepared by mixing 3.5 mg of L-Cysteine hydrochloride with 100 µL of DDI in a 1.5 ml microfuge tube, which was gently vortexed for 30 seconds until dissolved, providing a solution with a cysteine contentration of 200 mM. The 200 mM solution was diluted (with DDI) 1:20 to give a final concentration of 10 mM Cysteine solution. 40uL of the 10 mM Cysteine solution was added to the the 40uL of blood card sample, and the sample was incubated for 30 minutes on a vortex at room temperature to allow for reduction of all thiols.

Labelling with Mal-PEG 2000 for Reversible/Irreversible Analysis

The samples were removed from the vortex, and 80 µL of a 12.5 mM Methoxy polyethylene glycol 2000 solution (12.5 mM Methoxy polyethylene glycol 2000 in 40 mM Imidazole pH 7.4) was added to each sample, and the samples were incubated for 30 minutes on a vortex at room temperature.

Albumin isolation using Cibacron blue

5 µL aliquots of Cibacron blue were aliquoted into 0.5 mL microfuge tubes. 45 µL of 20 mM phosphate buffer was added, and gently mixed by flicking the tubes. The tubes were centrifuged for 1 minute, supernatant was removed and discarded. 40 µL of blood card disk solution and 160 µL of reduced blood card disk solution was added onto the Cibacron Blue, and gently mixed by flicking the tube. The tubes were incubated at room temperature for 10 minutes, and gently mixed by flicking the tubes. The tubes were centrigured for 1 minute, then the supernatant (containing un-bound whole proteins) was removed and discarded. 100 µL of 20 mM phosphate buffer was added to the Cibacron Blue-gel, and gently mixed before centrifuging then removing and discarding the supernatant to wash off any remaining unwanted whole blood components. The bound albumin was eluted by adding 25 µL of 1.4 M sodium chloride to the tube and gently mixing by flicking the tube. The tubes were centriguged, and the supernatant was removed and stored in a 0.5 mL microfuge tube. The store supernatant samples contained relatively purified albumin.

Gel Electrophoresis and Thiol Analysis

Purified albumin solution was mixed with equal volumes of sample buffer (i.e. 20 µl albumin solution with 20 µL of sample buffer). The samples were vortexed, and then 20 µL samples were loaded onto a 16% polyacrylamide gel. The gel was run at 180 V, 70 mA for 2 hours. The gel was imaged on ChemiDoc MP Imaging system using 5-minute exposure. Image J was used to quantify ratio of oxidised albumin to total albumin. The ratio of oxidized albumin to total albumin = [intensity of oxidized band /(intensity of reduced band + oxidized band)] x 100

**(e) Statistical analysis**

All data are presented as means $\pm$ SE unless otherwise states. Means were compared using a t-test or one-way ANOVA with repeated measures where appropriate. Significance was accepted at $p < 0.05$.

**2. Results**

Effect of exercise on reversibly oxidized albumin levels

A single participant performed a 5km run. Exercise intensity was modified by increasing the running speed and doubled between moderate and high intensity. Blood samples were taken 24hr after each run. A 2 day rest period was taken between the moderate and high intensity runs.

Figure 9 shows increases in the amount of reversibly oxidized albumin detected in the moderate and high inesnity exercise regimes compared to the baseline sample. Figure 9 also demonstrates a correlation between the intensity of the exercise perfomed, and the amount of reversibly oxidized albumin present in the blood sample.

Effect of inflammatory skin treatment on reversibly oxidized albumin levels

A female patient, aged 24, underwent medical microneedling treatment to the face. Blood samples were collected before treatment (baseline sample), and 24 hours after treatment was complete.

Figure 10 shows a marked (15%) increase in reversibly oxidized albumin post treatment compared to the baseline sample.

Effect of muscle damage on reversibly oxidized albumin levels

An untrained participant performed 4 sets of 6 repetitions (bicep curls) with a 5 kg weight. Blood samples were taken prior to performing the exercise and daily for 4 days post exercise. Figure 11 shows that the patient samples demonstrated high oxidative stress, indicative of sustained muscle damage. The patients oxidative stress profile had not recovered to pre-exercise levels at day 4.

Reversible and irreversible oxidation levels following exercise

A single participant performed two periods of a 4-day aerobic exercise trial. The two periods were separated by 2 weeks of rest. Exercise intensity (running duration and speed) were increased on day 2 and 4 with the highest intensity being on the 4th day of each exercise period. All samples were obrtained 24hr after exercise.

Figure 12 demonstrates large increases in the amount of reversably oxisised albumin measured in samples taken after both moderate and high intensity exercise. There was minimal corresponding change osbserved to levels of irreversibly oxidized albumin after either moderate or high intensity exercise.

Oxidative stress during sickness

A single male subject who was believed to be suffering from acute sinusitis. Samples were taken in the morning approximatly 24hr apart.

Figure 13 shows increases in both reversibly oxidized albumin and irreversibly oxidized albumin in pateints during periods of sickness.

Effect of aerobic exercise on reversibly oxidized albumin levels

Two patients with varying degress of aerobic fitness undertook an exercise program. Patient 1 was categorised as having good aerobic fitness, and Patient 2 was categorized as having poor aerobic fitness. The exercise program consisted of a 5km run, five twenty-minute soccer games, and a 100m sprint. Samples were taken from the patients before the exercise program began and upon completion of the program.

Figure 14 shows minimal difference between the levels of reversably oxidized albumin in Patient 1 pre and post exercise. There was a substantial increase in reversably oxidized albumin in Patient 2 post exercise.

Reversably oxidized albumin levels during muscle injury recovery

The Patient sustained a calf strain playing tennis. The patient complained that the muscle was very sore, and experienced significant loss of force. A physiotherapist confirmed a diagnosis of mucle injury and recommended a 1 to 2 week recovery period. Blood samples were taken from the patient regularly over a 10-day period beginning 24 hours post-injury.

Figure 15 shows a peak in the amount of reversably oxidized albumin at day 2 post injury. A steady decrease in the amount of reversably oxidized albumin was observed until day 10. The profile is consistent with the physiotherapists report and recommendation.

**Example 4 - Method of measuring relative oxidation levels of a protein using capillary electrophoresis**

**1. Materials/Methods**

**(a) Materials**

Double-deionized (DDI) water was used throughout. Protein molecular weight standards were purchased from Bio-Rad (Australia). Unless otherwise stated, all chemicals and reagents were obtained from Sigma-Aldrich (Castle Hill, Australia). Polyethylene glycol maleimide (malpeg), 5000 g/mol was purchased from JenKem Technology (USA).

**(b) Blood sample preparation and storage**

For the analysis of albumin thiol oxidation, nine parts of blood was collected into a $K_3$EDTA tube (Minicollect tubes $K_3$EDTA; Greiner Bio-One, Austria), containing 1 part of the trapping solution made up of 62.5 mM malpeg, 40 mM imidazole and 154 mM NaCl diluted in DDI water, pH 7.4. Additional blood was collected into a second $K_3$EDTA tube without trapping solution for the analysis of total plasma albumin. The tubes were briefly vortexed and then centrifuged (3000g, 10 min), and plasma collected. Plasma without trapping solution was immediately frozen in liquid nitrogen and stored at -80°C, whereas plasma with trapping solution was incubated at room temperature for 20 minutes and then frozen and stored.

**(c) Plasma sample preparation and storage**

Trapped plasma samples containing malpeg were thawed at 37°C with agitation and then divided into two 5$\mu$l aliquots.

Samples were prepared using the following protocols.

Sample 1 (trapped)- 5 $\mu$l of trapped-plasma (6.25mM PEG) was diluted with 490 $\mu$l of SDS/Tris buffer). 0.5uL of 100uM cysteine (200mM stock diluted 1/2 in DDI H20) was added. The sample is then placed on ice or stored at -80°C.

Sample 2 (trapped and reduced)- 5 $\mu$l of trapped-plasma (6.25mM PEG) was added to 5$\mu$l of 20 mM L cysteine (200mM stock diluted 1/10 in DDI H20). The sample was vortexed for 30 minutes to reduce reversibly oxidized albumin. 10uL of 25mM 10K PEG was then added and the sample was vortexed for 15 minutes to allow the PEG to bind to the albumin. 3uL of 100mM cysteine was added. Finally, 4.6 $\mu$l of the sample was diluted with 95 $\mu$l of SDS/Tris. The samples are then put on ice or stored at -80°C.

**(e) Capillary Electrophoresis under LabChip Protein Express protocol**

Samples were then loaded into LabChip GXII and run using the LabChip Protein Express protocol at an approximate albumin concentration of 0.023mg/ml.

**Results**

Figure 8A shows total albumin (A) and other blood proteins (B) in an untreated plasma sample. Figure 8B shows oxidised albumin (C) and reduced albumin (D) in a sample which was treated with malpeg. Figure 8C shows irreversibly oxidised albumin (E) and reversibly oxidised and reduced albumin (F) in a sample that underwent a first malpeg treatment step, a reduction step (with cysteine) and a second malpeg treatment step.

**References**

[1] U. Laemmli, SDS-page Laemmli method, Nature 227 (1970) 680-5.

[2] C.L. Ladner, J. Yang, R.J. Turner, R.A. Edwards, Visible fluorescent detection of proteins in polyacrylamide gels without staining, Analytical biochemistry 326(1) (2004) 13-20.

[3] C.A. Schneider, W.S. Rasband, K.W. Eliceiri, NIH Image to ImageJ: 25 years of image analysis, Nature methods 9(7) (2012) 671-675.

[4] L. Turell, H. Botti, L. Bonilla, M.J. Torres, F. Schopfer, B.A. Freeman, L. Armas, A. Ricciardi, B. Alvarez, R. Radi, HPLC separation of human serum albumin isoforms based on their isoelectric points, Journal of Chromatography B 944 (2014) 144-151

]5] Dayhoff-Brannigan, M., Ferrucci, L., Sun, K., Fried, L. P., Walston, J., Varadhan, R., Guralnik, J. M. and Semba, R. D. (2008). "Oxidative protein damage is associated with elevated serum interleukin-6 levels among older moderately to severely disabled women living in the community." The Journals of Gerontology Series A: Biological Sciences and Medical Sciences 63(2): 179-183.

[6] Matthaiou, C. M., Goutzourelas, N., Stages, D., Sarafoglou, E., Jamurtas, A., Koulocheri, S. D., Haroutounian, S. A., Tsatsakis, A. M. and Kouretas, D. (2014). "Pomegranate juice consumption increases GSH levels and reduces lipid and protein oxidation in human blood." Food and chemical toxicology 73: 1-6.

**Claims**

1. A method for assessing the oxidation states of a protein in a sample, wherein the protein is a protein selected from the list comprising: albumin, alpha-2-macroglobulin, fibrinogen beta chain, haptoglobin, immunoglobulin lambda constant 2, inter-alpha-trypsin inhibitor heavy chain H2, serotransferrin, immunoglobulin gamma-1 heavy chain, fibrinogen gamma chain and transthyretin, the method comprising the steps of:

   (a) contacting the sample with a first label comprising a sulfhydryl-reactive chemical group to selectively bind to a reduced cysteine group of the protein therein to form a first labelled sample;
   (b) forming a sub-sample of the first labelled sample;
   (c) treating the sub-sample with an effective amount of a thiol containing agent to cause a thiol-disulphide exchange that reduces at least one reversibly oxidised cysteine group of the protein therein preferentially to any irreversibly oxidised cysteine groups therein to form a treated sub-sample;
   (d) contacting the treated sub-sample with a second label comprising a sulfhydryl-reactive chemical group to selectively bind to a reduced cysteine group of the protein therein formed during step (c) to form a second labelled sample; and
   (e) assessing the first and second labelled samples for a plurality of oxidation states of the protein.

2. A method according to claim 1 wherein the protein is albumin and the at least one reversibly oxidised cysteine group is a reversibly oxidised cysteine group at cys34.

3. A method according to any one of the preceding claims wherein the first label is further adapted to trap the reduced cysteine group.

4. A method according to any one of the preceding claims wherein the first label is used at a concentration of at least 3mM, 3.6mM, 5mM, 6mM, 6.25mM, 7mM, 8mM, 9mM or 10mM.

5. A method according to any one of the preceding claims wherein the first label is contacted with the sample for at least 5, 10, 15 or 20 minutes.

6. A method according to any one of the preceding claims wherein the first label comprises a fluorescent compound.

7. A method according to any one of the preceding claims wherein the thiol containing agent is adapted to react with the reversibly oxidised cysteine group in a reaction with an equilibrium constant (K) value of between 1 and 2, 1 and 3 or 1 and 4.

8. A method according to any one of the preceding claims wherein the thiol containing agent is selected from the group comprising: cysteine, glutathione (reduced), mercaptoethanol, cysteamine, penicillamine and N-acetylcysteine.

9. A method according to any one of the preceding claims wherein the thiol containing agent is used at a final concentration of at least 2mM, 4mM, 6mM, 8mM, 10mM, 12mM, 12.5mM, 15mM or 20mM.

10. A method according to any one of the preceding claims wherein the thiol containing agent is contacted with the subsample for at least 5, 10, 15, 20 or 30 minutes.

11. A method according to any one of the preceding claims further comprising the step of quantifying the amount of the identified oxidation states of the protein.

**Patentansprüche**

1. Verfahren zum Bewerten der Oxidationszustände eines Proteins in einer Probe, wobei das Protein ein Protein ist, das aus der Liste ausgewählt ist, die Folgendes umfasst: Albumin, Alpha-2-Makroglobulin, Fibrinogen-Beta-Kette, Haptoglobin, Immunglobulin Lambda-Konstante 2, Inter-Alpha-Trypsin Inhibitor der schweren Kette H2, Serotransferrin, schwere Kette des Immunglobulins Gamma-1, Fibrinogen-Gammakette und Transthyretin, wobei das Verfahren die Schritte umfasst zum:

(a) Inkontaktbringen der Probe mit einer ersten Markierung, die eine Sulfhydryl-reaktive chemische Gruppe umfasst, um selektiv an eine reduzierte Cysteingruppe des Proteins darin zu binden, um eine erste markierte Probe zu bilden;

(b) Bilden einer Teilprobe der ersten markierten Probe;

(c) Behandeln der Teilprobe mit einer wirksamen Menge eines thiolhaltigen Mittels, um einen Thiol-Disulfid-Austausch zu veranlassen, der mindestens eine reversibel oxidierte Cysteingruppe des Proteins darin bevorzugt gegenüber allen irreversibel oxidierten Cysteingruppen darin reduziert, um eine behandelte Teilprobe zu bilden;

(d) Inkontaktbringen der behandelten Teilprobe mit einer zweiten Markierung, die eine Sulfhydryl-reaktive chemische Gruppe umfasst, um selektiv an eine reduzierte Cysteingruppe des darin im Schritt (c) gebildeten Proteins zu binden, um eine zweite markierte Probe zu bilden; und

(e) Bewerten der ersten und zweiten markierten Proben auf eine Vielzahl von Oxidationszuständen des Proteins.

2. Verfahren nach Anspruch 1, wobei das Protein Albumin ist und die mindestens eine reversibel oxidierte Cysteingruppe eine reversibel oxidierte Cysteingruppe an cys34 ist.

3. Verfahren nach einem der vorstehenden Ansprüche, wobei die erste Markierung weiter angepasst ist, um die reduzierte Cysteingruppe abzufangen.

4. Verfahren nach einem der vorstehenden Ansprüche, wobei die erste Markierung in einer Konzentration von mindestens 3 mM, 3,6 mM, 5 mM, 6 mM, 6,25 mM, 7 mM, 8 mM, 9 mM oder 10 mM verwendet wird.

5. Verfahren nach einem der vorstehenden Ansprüche, wobei die erste Markierung mindestens 5, 10, 15 oder 20 Minuten lang mit der Probe in Kontakt gebracht wird.

6. Verfahren nach einem der vorstehenden Ansprüche, wobei die erste Markierung eine fluoreszierende Verbindung umfasst.

7. Verfahren nach einem der vorstehenden Ansprüche, wobei das thiolhaltige Mittel angepasst ist, um mit der reversibel oxidierten Cysteingruppe in einer Reaktion mit einem Gleichgewichtskonstantenwert (K) zwischen 1 und 2, 1 und 3 oder 1 und 4 zu reagieren.

8. Verfahren nach einem der vorstehenden Ansprüche, wobei das thiolhaltige Mittel aus der Gruppe ausgewählt ist, die umfasst: Cystein, Glutathion (reduziert), Mercaptoethanol, Cysteamin, Penicillamin und N-Acetylcystein.

9. Verfahren nach einem der vorstehenden Ansprüche, wobei das thiolhaltige Mittel in einer Endkonzentration von mindestens 2 mM, 4 mM, 6 mM, 8 mM, 10 mM, 12 mM, 12,5 mM, 15 mM oder 20 mM verwendet wird.

10. Verfahren nach einem der vorstehenden Ansprüche, wobei das thiolhaltige Mittel mit der Teilprobe mindestens 5, 10, 15, 20 oder 30 Minuten lang in Kontakt gebracht wird.

11. Verfahren nach einem der vorstehenden Ansprüche, das weiter den Schritt der Quantifizierung der Menge der identifizierten Oxidationszustände des Proteins umfasst.

**Revendications**

1. Procédé pour évaluer les états d'oxydation d'une protéine dans un échantillon, dans lequel la protéine est une protéine sélectionnée dans la liste comprenant : albumine, alpha-2-macroglobuline, chaîne bêta de fibrinogène, haptoglobine, constante lambda 2 d'immunoglobuline, chaîne lourde d'inhibiteur d'inter-alpha-trypsine H2, sérotransferrine, chaîne lourde d'immunoglobuline gamma-1, chaîne gamma de fibrinogène et transthyrétine, le procédé comprenant les étapes consistant à :

(a) mettre l'échantillon en contact avec un premier marqueur comprenant un groupe chimique réactif au sulfhydryle pour une liaison sélective à un groupe cystéine réduit de la protéine en son sein afin de former un premier échantillon marqué ;

(b) former un sous-échantillon du premier échantillon marqué ;

(c) traiter le sous-échantillon avec une quantité efficace d'un agent contenant un thiol pour provoquer un échange thiol-disulfure qui réduit au moins un groupe cystéine oxydé de manière réversible de la protéine en son sein, de préférence à tout groupe cystéine oxydé de manière irréversible en son sein pour former un sous-échantillon traité ;

(d) mettre le sous-échantillon traité en contact avec un second marqueur comprenant un groupe chimique réactif au sulfhydryle pour une liaison sélective à un groupe cystéine réduit de la protéine en son sein formée au cours de l'étape (c) afin de former un second échantillon marqué ; et

(e) évaluer les premier et second échantillons marqués pour une pluralité d'états d'oxydation de la protéine.

2. Procédé selon la revendication 1, dans lequel la protéine est l'albumine et le au moins un groupe cystéine oxydé de manière réversible est un groupe cystéine oxydé de manière réversible en cys34.

3. Procédé selon l'une quelconque des revendications précédentes, dans lequel le premier marqueur est en outre adapté pour piéger le groupe cystéine réduit.

4. Procédé selon l'une quelconque des revendications précédentes, dans lequel le premier marqueur est utilisé à une concentration d'au moins 3 mM, 3,6 mM, 5 mM, 6 mM, 6,25 mM, 7 mM, 8 mM, 9 mM ou 10 mM.

5. Procédé selon l'une quelconque des revendications précédentes, dans lequel le premier marqueur est mis en contact avec l'échantillon pendant au moins 5, 10, 15 ou 20 minutes.

6. Procédé selon l'une quelconque des revendications précédentes, dans lequel le premier marqueur comprend un composé fluorescent.

7. Procédé selon l'une quelconque des revendications précédentes, dans lequel l'agent contenant un thiol est adapté pour réagir avec le groupe cystéine oxydé de manière réversible dans une réaction avec une valeur de constante d'équilibre (K) comprise entre 1 et 2, 1 et 3 ou 1 et 4.

8. Procédé selon l'une quelconque des revendications précédentes, dans lequel l'agent contenant un thiol est choisi dans le groupe comprenant : la cystéine, le glutathion (réduit), le mercaptoéthanol, la cystéamine, la pénicillamine et la N-acétylcystéine.

9. Procédé selon l'une quelconque des revendications précédentes, dans lequel l'agent contenant un thiol est utilisé à une concentration finale d'au moins 2 mM, 4 mM, 6 mM, 8 mM, 10 mM, 12 mM, 12,5 mM, 15 mM ou 20 mM.

10. Procédé selon l'une quelconque des revendications précédentes, dans lequel l'agent contenant un thiol est mis en contact avec le sous-échantillon pendant au moins 5, 10, 15, 20 ou 30 minutes.

11. Procédé selon l'une quelconque des revendications précédentes, comprenant en outre l'étape consistant à quantifier la quantité des états d'oxydation identifiés de la protéine.

**Figure 1**

**Figure 2**

**Figure 3**

## Figure 4

A

B

C

## Figure 5

# Figure 6

# Figure 7

## Figure 8A

## Figure 8B

## Figure 8C

## Figure 9

## Figure 10

## Figure 11

## Figure 12

**Figure 13**

**Day Post Sickness**

**Figure 14**

## Figure 15

## Figure 16

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **BURGOYNE JOSEPH ROBERT et al.** *JOURNAL OF PHARMACOLOGICAL AND TOXICOLOGICAL METHODS,* vol. 68 (3), 297-301
- **ANTONIO JUNIOR LEPEDDA et al.** *OXIDATIVE MEDICINE AND CELLULAR LONGEVITY,* vol. 30 (8), 2113-7
- **BAR-OR DAVID et al.** *CLINIC A CHIMICA ACTA,* vol. 387 (1), 120-127
- **U. LAEMMLI.** SDS-page Laemmli method. *Nature,* 1970, vol. 227, 680-5
- **C.L. LADNER ; J. YANG ; R.J. TURNER ; R.A. EDWARDS.** Visible fluorescent detection of proteins in polyacrylamide gels without staining. *Analytical biochemistry,* 2004, vol. 326 (1), 13-20
- **C.A. SCHNEIDER ; W.S. RASBAND ; K.W. ELICEIRI.** NIH Image to ImageJ: 25 years of image analysis. *Nature methods,* 2012, vol. 9 (7), 671-675
- **L. TURELL ; H. BOTTI ; L. BONILLA ; M.J. TORRES ; F. SCHOPFER ; B.A. FREEMAN ; L. ARMAS ; A. RICCIARDI ; B. ALVAREZ ; R. RADI.** HPLC separation of human serum albumin isoforms based on their isoelectric points. *Journal of Chromatography,* 2014, vol. 944, 144-151
- **DAYHOFF-BRANNIGAN, M. ; FERRUCCI, L. ; SUN, K. ; FRIED, L. P. ; WALSTON, J. ; VARADHAN, R. ; GURALNIK, J. M. ; SEMBA, R. D.** Oxidative protein damage is associated with elevated serum interleukin-6 levels among older moderately to severely disabled women living in the community. *The Journals of Gerontology Series A: Biological Sciences and Medical Sciences,* 2008, vol. 63 (2), 179-183
- **MATTHAIOU, C. M. ; GOUTZOURELAS, N. ; STAGES, D. ; SARAFOGLOU, E. ; JAMURTAS, A. ; KOULOCHERI, S. D. ; HAROUTOUNIAN, S. A. ; TSATSAKIS, A. M. ; KOURETAS, D.** Pomegranate juice consumption increases GSH levels and reduces lipid and protein oxidation in human blood. *Food and chemical toxicology,* 2014, vol. 73, 1-6